# EUROPEAN PATENT SPECIFICATION

(11) **EP 3 835 417 B1**
(45) Date of publication and mention of the grant of the patent: **06.05.2026**
(21) Application number: 19846321.8
(22) Date of filing: 08.08.2019
(51) Int. Cl.: C12N 5/09, C12Q 1/02, G01N 33/15, G01N 33/50, C12N 5/00

(54) **METHOD FOR CULTURING CANCER TISSUE OR TISSUE ANALOGOUS TO CANCER TISSUE**
VERFAHREN ZUR KULTIVIERUNG VON KREBSGEWEBE ODER GEWEBE ANALOG ZU KREBSGEWEBE
PROCÉDÉ POUR LA CULTURE DE TISSU CANCÉREUX OU DE TISSU ANALOGUE À DU TISSU CANCÉREUX

(30) Priority: 08.08.2018 JP 2018149047
(43) Date of publication of application: 16.06.2021
(73) Proprietor: CHUGAI SEIYAKU KABUSHIKI KAISHA, Tokyo 115-8543 (JP)
(72) Inventor: KAWAI, Shigeto, Tokyo 153-8904 (JP); YAMAZAKI, Masaki, Gotemba-shi, Shizuoka 412-8513 (JP); NAKANO, Kiyotaka, Tokyo 153-8904 (JP); SUZUKI, Masami, Tokyo 153-8904 (JP); YAMAZAKI, Tatsumi, Tokyo 103-8324 (JP)
(74) Representative: Vossius & Partner Patentanwälte Rechtsanwälte mbB
(86) International application number: PCT/JP2019/031328
(87) International publication number: WO 2020/032162

(56) References cited:
- EP-A1- 2 772 268
- WO-A1-2013/035824
- WO-A1-2017/207737
- JP-A- 2012 254 081
- JP-A- 2014 204 674
- JP-A- 2018 029 583
- JP-A- 2018 110 575
- CARLA S VERISSIMO ET AL: "Targeting mutant RAS in patient-derived colorectal cancer organoids by combinatorial drug screening", ELIFE, vol. 5, 15 November 2016 (2016-11-15), XP055508797, DOI: 10.7554/eLife.18489
- SHINTA KOBAYASHI ET AL: "LGR5-Positive Colon Cancer Stem Cells Interconvert with Drug-Resistant LGR5-Negative cells and are Capable of Tumor Reconstitution", STEM CELLS, vol. 30, no. 12, 1 December 2012 (2012-12-01), pages 2631 - 2644, XP055050272, ISSN: 1066-5099, DOI: 10.1002/stem.1257
- BENJAMIN L EMMINK ET AL: "Differentiated Human Colorectal Cancer Cells Protect Tumor-Initiating Cells From Irinotecan", GASTROENTEROLOGY, ELSEVIER INC, US, vol. 141, no. 1, 15 March 2011 (2011-03-15), pages 269 - 278, XP028296749, ISSN: 0016-5085, [retrieved on 20110401], DOI: 10.1053/J.GASTRO.2011.03.052
- BUZZELLI JON N. ET AL: "Colorectal cancer liver metastases organoids retain characteristics of original tumor and acquire chemotherapy resistance", vol. 27, 28 January 2018 (2018-01-28), NL, pages 109 - 120, XP055906285, ISSN: 1873-5061, Retrieved from the Internet <URL:https://www.sciencedirect.com/science/article/pii/S1873506118300229/pdfft?md5=1132b5ce6604902a2a44838e15f20f80&pid=1-s2.0-S1873506118300229-main.pdf> DOI: 10.1016/j.scr.2018.01.016
- VAN DE WETERING MARC ET AL: "Prospective Derivation of a Living Organoid Biobank of Colorectal Cancer Patients", CELL, ELSEVIER, AMSTERDAM NL, vol. 161, no. 4, 7 May 2015 (2015-05-07), pages 933 - 945, XP029224300, ISSN: 0092-8674, DOI: 10.1016/J.CELL.2015.03.053
- NAGLE PETER W ET AL: "Patient-derived tumor organoids for prediction of cancer treatment response", SEMINARS IN CANCER BIOLOGY, SAUNDERS SCIENTIFIC PUBLICATIONS, PHILADELPHIA, PA, US, vol. 53, 30 June 2018 (2018-06-30), pages 258 - 264, XP085546204, ISSN: 1044-579X, DOI: 10.1016/J.SEMCANCER.2018.06.005
- FUJII ETSUKO ET AL: "A simple method for histopathological evaluation of organoids", vol. 31, no. 1, 24 November 2017 (2017-11-24), JP, pages 81 - 85, XP055906468, ISSN: 0914-9198, Retrieved from the Internet <URL:https://www.ncbi.nlm.nih.gov/pmc/articles/PMC5820108/pdf/tox-31-081.pdf> DOI: 10.1293/tox.2017-0060
- KAWAI SHIGETO ET AL: "Three-dimensional culture models mimic colon cancer heterogeneity induced by different microenvironments", vol. 10, no. 1, 21 February 2020 (2020-02-21), pages 3156, XP055906268, Retrieved from the Internet <URL:https://www.nature.com/articles/s41598-020-60145-9.pdf> DOI: 10.1038/s41598-020-60145-9
- HOFFMANN O. I. ET AL.: "Impact of the spheroid model complexity on drug response", JOURNAL OF BIOTECHNOLOGY, vol. 205, 2015, pages 14 - 23, XP029240262, ISSN: 0168-1656, DOI: 10.1016/j.jbiotec.2015.02.029

## Description

### [Technical Field]

Provided are methods for culturing a a tissue analogous to a cancer tissue, cell clusters obtained by the culturing methods, and their utilization. In particular, the present invention relates to a culture method for forming a treatment resistant organoid, wherein the method comprises a step of three-dimensionally culturing an organoid which was treated with at least one anticancer agent and isolating a treatment resistant organoid that is resistant to the treatment with said anticancer agent, wherein the step of three-dimensionally culturing is culturing in the absence of said at least one anticancer agent and wherein the organoid is formed by a pre-culture step of culturing one or more cancer stem cells.

### [Background Art]

The number of deaths due to cancer continues to increase worldwide. For example, cancer has become the leading cause of death of Japanese people in Japan as well. For some cancers, screenings for early cancer detection and the spread of vaccines for preventing viral infections, which are cancer risk factors, are showing improvements in treatment results and preventive effects. Furthermore, application of new technologies is being tried out one after another in cancer treatments using anticancer agents, surgical procedures, or irradiation with radiation or proton beams. Among these various cancer treatment techniques, the use of anticancer agents is still one of the major options. In the treatment of cancer with anticancer drugs (chemotherapy), acquisition of anticancer agent resistance by cancers is one of the major causes hindering treatment, and elucidation of the mechanism is awaited.

In the treatment of colorectal cancer for example, treatment efficiency has been enhanced through improvements in new agents and surgical excision methods. As with other organs, the so-called neoadjuvant therapy or conversion therapy, where a tumor is shrunk by drug therapy in advance and then targeted for surgery, has become widely used even for cancers that are difficult to be excised. Advances in treatment technologies have also increased the number of cases of cancers that were previously not subjected to surgical excision, but are now being treated. However, there are still many cases of recurrence despite the success in treatments. One of the causes of recurrence is considered to be the acquisition of anticancer agent resistance by cancers. As for the reason for resistance, it is assumed that anticancer agent treatment induces a group of cells that have acquired resistance and that manage to survive. However, regarding the origin of cells that acquire resistance, several different theories such as cancer stem cells (CSC) and epithelial mesenchymal transition (EMT) have been advocated, but there is still a great deal of ongoing debate. In addition, there are no research reports that have captured the substance of resistance in actual clinical settings. Understanding this mechanism is important in order to regulate the acquisition of anticancer agent resistance by cancer cells.

The present inventors have previously obtained a specific antibody against LGR5 (leucine-rich repeat-containing G-protein-coupled receptor 5), which is a CSC marker for colorectal cancer, demonstrated that CSC can be detected with high sensitivity in clinical tissues, and also established a CSC cell line (NPL 1,NPL 2 and PTL 3).

Furthermore, an attempt to culture CSC by combining various growth factors is known (PTL 1). A method of culturing epithelial stem cells in an extracellular matrix also containing a growth factor has also been proposed (PTL 2). However, these known CSCs and their culture techniques are limited to three-dimensional culture of CSCs, and the mechanism of how cancers acquire resistance to anticancer agents was not elucidated.

### [Citation List]

### [Patent Literature]

[PTL 1] WO2012/168930
[PTL 2] US2012/0196312
[PTL 3] WO2013/062083

### [Non-Patent Literature]

[NPL 1] Kobayashi, S., et al. Stem cells 30, 2631-2644 (2012).
[NPL 2] Yamazaki, M., et al. Acta histochemica et cytochemica 48, 159-164 (2015)

### [Summary of Invention]

### [Technical Problem]

An objective of the present disclosure is to provide a technique that enables the construction of a model of cancer tissue that has acquired anticancer agent resistance. Alternatively, an objective of the present disclosure is to use the model to elucidate the process of acquiring anticancer agent resistance in a cancer tissue, and to provide a new cancer treating technique that targets a target molecule in an anticancer agent-resistant cancer tissue or a method for discovering a cancer treating technique that overcomes the resistance of a cancer to an anticancer agent.

### [Solution to Problem]

The present inventors discovered that small cell clusters are formed in cultures when a cancer tissue or a tissue analogous to a cancer tissue which was treated with an anticancer agent is three-dimensionally cultured, and elucidated the properties thereof. The newly discovered small cell clusters resemble the survival pattern of cancer cells in a patient's body when treated with an anticancer agent, which are found in cancer tissues removed from cases in which chemotherapy has been extremely effective. Furthermore, they found that the cells in the small cell clusters found in the culture were similar to surviving cancer cells in a patient's body when treated with an anticancer agent in terms of CSC phenotype and mRNA expression level. Thus, the present inventors succeeded in artificially regenerating, as a model of an anticancer agent-resistant cancer tissue, treatment-resistant cell clusters having the characteristics of surviving cancer cells during treatment with an anticancer agent in a patient's cancer tissue, and completed the present disclosure.

To date, there have been reports of the establishment of organoids from cancer tissues (J N. Buzzelli et al., Stem Cell Res (2018) 27: 109-120, C. S Verissimo et al., Elife (2016) 5. Pii. : e18489, Emmink B. et al. Gastroenterology (2011) 141:269-278, van de Wetering et al., (2015), Cell 161, 933-945, Nagle, P.W. et al., Seminars in Cancer Biology 53 (2018) 258-264, Fujii, E et al., J Toxicol Pathol (2018); 31: 81-85). However, there are no known examples of attempting three-dimensional culture after anticancer agent treatment.

The invention is directed to the embodiments as set forth in the claims. In particular, the invention relates to a culture method for forming a treatment resistant organoid, wherein the method comprises a step of three-dimensionally culturing an organoid which was treated with at least one anticancer agent and isolating a treatment resistant organoid that is resistant to the treatment with said anticancer agent, wherein the step of three-dimensionally culturing is culturing in the absence of said at least one anticancer agent and wherein the organoid is formed by a pre-culture step of culturing one or more cancer stem cells.
The present description frequently references the term "cell cluster". In the context of the invention, the term cell cluster is understood to refer to an organoid.

Through this disclosure, the present inventors succeeded in constructing a model that highly reproduces cancer tissues that have acquired anticancer agent resistance. The present disclosure makes it possible to elucidate the mechanism of a cancer to acquire anticancer agent resistance and to search for therapeutic strategies for anticancer agent resistant cancers.

### [Brief Description of Drawings]

Fig. 1 shows a method for producing organoids.
Fig. 2 shows the proliferation of organoids when cells are three-dimensionally cultured in Matrigel.
Fig. 3 shows the results of gene expression analysis of organoids.
Fig. 4 shows the structure of organoids and marker expression.
Fig. 5 shows a method for producing regrowing organoids.
Fig. 6 is photographs of microscopic observation of regrowing organoids.
Fig. 7 shows the structure of regrowing organoids.
Fig. 8 shows the results of FACS analysis of LGR5 expression in regrowing organoids.
Fig. 9 shows the structure of regrowing organoids and marker expression.
Fig. 10 shows the results of gene expression analyses of ROS metabolism-associated genes.
Fig. 11 shows the structure of organoids and the expression of GPX1 and GPX2 proteins.
Fig. 12 shows a method of adding an inhibitor to regrowing organoids.
Fig. 13 shows the growth of organoids supplemented with the gamma-glutamylcysteine synthetase inhibitor L-buthionine-sulfoximine and/or the thioredoxin reductase inhibitor auranofin during re-culture.

### [Description]

In the present disclosure, a culture method comprising a culture step of three-dimensionally culturing a cancer tissue or a tissue analogous to a cancer tissue which was treated with an anticancer agent is provided. Cancer cells having resistance to the anticancer agent can be obtained through the culture method of the present disclosure.

### Method of culturing a cancer tissue or a tissue analogous to a cancer tissue

As the first aspect of the present disclosure, a method for culturing a cancer tissue or a tissue analogous to a cancer tissue is provided.

The cancer tissue or tissue analogous to the cancer tissue cultured by the culture method of the present disclosure is a tissue treated with an anticancer agent.

### Anticancer agent

In the present specification, any agent that acts on cancer cells can be used as the "anticancer agent". Examples include an agent that inhibits the growth of cancer cells, an agent that enhances the action of immunocompetent cells on cancer cells, and the like. The inhibiting action of cancer cell growth includes inhibition of protein and nucleic acid synthesis, inhibition of signaling of cancer cell growth factors, so-called molecular target inhibition, and inhibition of cancer cell growth factors such as various hormones (antagonists), etc. When the anticancer agent is a chemically-synthesized molecule, it is especially called a chemotherapeutic agent. Therapeutic agents for diseases include not only synthetic drugs, but also biological formulations (biopharmaceuticals) such as antibodies and cytokines. Variants of antibodies and cytokines whose action is artificially enhanced and variants with improved blood kinetics are also called biological formulations. Antibodies include monoclonal antibodies, chimeric antibodies, humanized antibodies, and the like. Furthermore, cells that act on cancer cells, such as cytotoxic T cells, can also be used as therapeutic agents for diseases. Cytotoxic T cells also include so-called CAR (Chimeric Antigen Receptor)-T cells, in which the receptor has been introduced by gene recombination technology.

The anticancer agent used in the present disclosure may be any available compound. Anticancer agents that have already been clinically applied can reconstitute the treatment-resistant cell clusters that actually occur in patients. Alternatively, when a candidate agent under development is used, it will be possible to know whether the agent can produce a treatment-resistant cell cluster.

### Chemotherapeutic agents

"Chemotherapeutic agent" refers to a chemical compound useful for the treatment of cancer. Examples of chemotherapeutic agents include: alkylating agents such as thiotepa and cyclophosphamide (CYTOXAN^{®}); alkyl sulfonates such as busulfan, improsulfan, and piposulfan; aziridines such as benzodopa, carboquone, meturedopa, and uredopa; ethyleneimines and methylolmelamines including altretamine, triethylenemelamine, triethylene phosphoramide, triethylene thiophosphoramide, and trimethylomelamine; acetogenins (especially bullatacin and bullatacinone); delta-9-tetrahydrocannabinol (dronabinol, MARINOL^{®}); beta-lapachone; lapachol; colchicines; betulinic acid; camptothecin (including the synthetic analogue topotecan (HYCAMTIN^{®}), CPT-11 (irinotecan, CAMPTOSAR^{®}), acetyl camptothecin, scopolectin, and 9-amino camptothecin); bryostatin; callystatin; CC-1065 (including its adozelesin, carzelesin, and bizelesin synthetic analogues); podophyllotoxin; podophyllic acid; teniposide; cryptophycins (particularly cryptophycin 1 and cryptophycin 8); dolastatin; duocarmycin (including the synthetic analogues KW-2189 and CB1-TM1); eleutherobin; pancratistatin; sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterin, prednimustine, trofosfamide, and uracil mustard; nitrosoureas such as carmustine, chlorozotocin, fotemustine, lomustine, nimustine, and ranimustine; antibiotics such as enediyne antibiotics {e.g., calicheamicin, especially calicheamicin gamma 1I and calicheamicin omega I1 (for example, see Nicolaou. et al., Angew. Chem Intl. Ed. Engl., 33: 183-186 (1994)); the oral alpha-4 integrin inhibitor CDP323; dinemycins including dinemycin A; esperamicin; and neocarzinostatin chromophore and related chromoprotein enediyne antibiotic chromophores}, aclacinomycin, actinomycin, anthramycin, azaserine, bleomycin, cactinomycin, carubicin, carminomycin, carzinophilin, chromomycin, dactinomycin, daunorubicin, detorubicin, 6-diazo-5-oxo-L-norleucine, doxorubicin (including ADRIAMYCIN^{®}, morpholino-doxorubicin, cyanomorpholino-doxorubicin, 2-pyrrolino-doxorubicin, doxorubicin HCl liposome injection (DOXIL^{®}), liposome doxorubicin TCL D-99 (MYOCET^{®}), pegylated liposome doxorubicin (CAELYX^{®}), and deoxydoxorubicin), epirubicin, esorubicin, idarubicin, marcellomycin, mitomycins such as mitomycin C, mycophenolic acid, nogalamycin, olivomycin, peplomycin, porfiromycin, puromycin, quelamycin, rodorubicin, streptonigrin, streptozocin, tubercidin, ubenimex, zinostatin, and zorubicin; antimetabolites such as methotrexate, gemcitabine (GEMZAR^{®}), tegafur (UFTORAL^{®}), capecitabine (XELODA^{®}), epothilone, and 5-fluorouracil (5-FU); folic acid analogs such as denopterin, methotrexate, pteropterin, and trimetrexate; purine analogs such as fludarabine, 6-mercaptopurine, thiamiprine, and thioguanine; pyrimidine analogs such as ancitabine, azacytidine, 6-azauridine, carmofur, cytarabine, dideoxyuridine, doxifluridine, enocitabine, and floxuridine; androgens such as calusterone, dromostanolone propionate, epithiostanol, mepitiostane, and testolactone; anti-adrenals such as aminoglutethimide, mitotane, and trilostane; folic acid supplements such as folinic acid; aceglatone; aldophosphamide glycoside; aminolevulinic acid; eniluracil; amsacrine; bestrabucil; bisantrene; edatrexate; defofamine; demecolcine; diaziquone; elflornithine; elliptinium acetate; epothilone; etoglucid; gallium nitrate; hydroxyurea; lentinan; lonidamine; maytansinoids such as maytansine and ansamitocin; mitoguazone; mitoxantrone; mopidamol; nitraerine; pentostatin; phenamet; pirarubicin; losoxantrone; 2-ethylhydrazide; procarbazine; PSK^{®} polysaccharide complex (JHS Natural Products, Eugene, OR); razoxane; rhizoxin; schizophyllan; spirogermanium; tenuazonic acid; triaziquone; 2,2',2'-trichloro triethylamine; trichothecenes (particularly T-2 toxin, verracurin A, roridin A, and anguidine); urethane; vindesine (ELDISINE^{®}, FILDESIN^{®}); dacarbazine; mannomustine; mitobronitol; mitolactol; pipobroman; gacytosine; arabinoside ("Ara-C"); thiotepa; taxoids such as paclitaxel (TAXOL^{®}), albumin-modified nanoparticle formulation of paclitaxel (ABRAXANE^{®}), and docetaxel (TAXOTERE^{®}); chlorambucil; 6-thioguanine; mercaptopurine; methotrexate; platinum agents such as cisplatin, oxaliplatin (e.g. ELOXATIN^{®}), and carboplatin; vincas which prevent the formation of microtubules by tubulin polymerization, including vinblastine (VELBAN^{®}), vincristine (ONCOVIN^{®}), vindesine (ELDISINE^{®}, FILDESIN^{®}), and vinorelbine (NAVELBINE^{®}); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid including bexarotene (TARGRETIN^{®}); bisphosphonates such as clodronate (e.g., BONEFOS^{®} or OSTAC^{®}) , etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}); or risedronate (ACTONEL^{®}); troxacitabine (a 1,3-dioxolane nucleoside cytosine analog); antisense oligonucleotides, especially those that inhibit the expression of genes in signaling pathways associated with aberrant cell proliferation, such as PKC-alpha, Raf, H-Ras, and epidermal growth factor receptor (EGF-R); vaccines such as THERATOPE^{®} vaccines and gene therapy vaccines (e.g., ALLOVECTIN^{®} vaccine, LEUVECTIN^{®} vaccine, and VAXID^{®} vaccine); topoisomerase 1 inhibitors (e.g., LURTOTECAN^{®}); rmRH (e.g., ABARELIX^{®}); BAY439006 (sorafenib; Bayer); SU-11248 (sunitinib, SUTENT^{®}, Pfizer); perifosine, COX-2 inhibitors (e.g., celecoxib or etoricoxib), proteosome inhibitors (e.g., PS341); bortezomib (VELCADE^{®}); CCI-779; tipifarnib (R11577); sorafenib, ABT510; Bcl-2 inhibitors such as oblimersen sodium (GENASENSE^{®}); pixantrone; EGFR inhibitors (see definition below); tyrosine kinase inhibitors (see definition below); serine-threonine kinase inhibitors such as rapamycin (sirolimus, RAPAMUNE^{®}); farnesyltransferase inhibitors such as lonafarnib (SCH 6636 , SARASAR ^{™}); and pharmaceutically acceptable salts, acids, or derivatives of any of the above; and combinations of two or more of the above such as CHOP, an abbreviation for the combination therapy of cyclophosphamide, doxorubicin, vincristine, and prednisolone; and FOLFOX, an abbreviation for a treatment regimen where oxaliplatin (ELOXANTIN ^{™}) is combined with 5-FU and leucovorin.

Chemotherapeutic agents as defined herein include "antihormonal agents" or "endocrine therapeutic agents" which act to regulate, reduce, block, or inhibit the effects of hormones that can promote cancer growth. They may be hormones themselves and include, but are not limited to: anti-estrogens with a mixed agonist/antagonist profile including tamoxifen (NOLVADEX^{®}), 4-hydroxytamoxifen, toremifene (FARESTON^{®}), idoxifene, droloxifene, raloxifene (EVISTA^{®}), trioxifene, keoxifene, and selective estrogen receptor modulators (SERMs) such as SERM3; pure anti-estrogens without agonist properties such as fulvestrant (FASLODEX^{®}) and EM800 (such agents may block estrogen receptor (ER) dimerization, inhibit DNA binding, increase ER turnover, and/or suppress ER levels); aromatase inhibitors including steroidal aromatase inhibitors such as formestane and exemestane (AROMASIN^{®}), and nonsteroidal aromatase inhibitors such as anastrozole (ARIMIDEX^{®}), letrozole (FEMARA^{®}), and aminoglutethimide, as well as other aromatase inhibitors including vorozole (RIVISOR^{®}), megestrol acetate (MEGASE^{®}), fadrozole, and 4(5)-imidazole; luteinizing hormone-releasing hormone agonists including leuprolide (LUPRON^{®} and ELIGARD^{®}), goserelin, buserelin, and triptorelin; sex steroids including progestins such as megestrol acetate and medroxyprogesterone acetate, estrogens such as diethylstilbestrol and premarin, and androgens/retinoids such as fluoxymesterone, all-trans retinoic acid, and fenretinide; onapristone; anti-progesterones; estrogen receptor down-regulators (ERDs); anti-androgens such as flutamide, nilutamide, and bicalutamide; and pharmaceutically acceptable salts, acids, or derivatives of any of the above; and combinations of two or more of the above.

### Anticancer agent treatment

In the present disclosure, a cancer tissue or a tissue analogous to cancer tissue is treated with an anticancer agent in advance before being three-dimensionally cultured. For example, when a cancer tissue or a tissue analogous to a cancer tissue is brought into contact with an anticancer agent, it can be treated with the anticancer agent. When artificially forming a tissue analogous to a cancer tissue, for example, when preparing it by culturing from cancer stem cells, the cancer stem cells are cultured under appropriate conditions, and the tissue is formed in a few days to ten days. Then, an anticancer agent can be added to the culture system, and the tissue formed from the cancer stem cells can be treated with the anticancer agent. As to the time of treatment with the anticancer agent, conditions appropriate for obtaining the cancer tissue or tissue analogous to cancer tissue, which is the ultimate target of culture, can be selected according to the combination of the anticancer agent and the cancer tissue or tissue analogous to the cancer tissue, and such. For example, the cancer tissue or tissue analogous to the cancer tissue can be treated with an anticancer agent for one day to one week.

A tissue can be treated with a single anticancer agent, or with a combination of different types of anticancer agents. For example, a combination of anticancer agents that are administered in combination in actual clinical settings is useful as a combination of anticancer agents for treating the tissue. In addition, the anticancer agent may be a compound administered to a living body or a derivative such as a metabolite thereof, as long as it can inhibit the growth of cancer cells. When the therapeutic agents for diseases does not easily dissolve in the culture solution, a dissolving aid can be used. For example, dimethyl sulfoxide (DMSO) is widely used as a dissolving aid with low cytotoxicity and excellent miscibility with water.

It is desirable that anticancer agents are brought into contact with cancer stem cell-derived tissues under conditions that allow each anticancer agent to act on the cancer. Therefore, when an auxiliary factor is required to obtain the anticancer effect of an anticancer agent, it can be contacted with the tissue together with the necessary factor. For example, the cytotoxic action of an antibody may require various cytokines, complements, predatory cells, and the like.

### Three-dimensional culture after anticancer agent treatment

The present disclosure includes a step of further three-dimensionally culturing a cancer tissue or a tissue analogous to a cancer tissue which was treated with an anticancer agent. For example, in an adherent culture, cells usually proliferate in a two-dimensional direction on a plane, whereas in a three-dimensional culture, cell formation in a three-dimensional direction is encouraged. Specifically, a three-dimensional culture is possible by culturing a cancer stem cell-derived tissue in a liquid or extracellular matrix. In particular, when the cancer stem cell-derived tissue is retained in an extracellular matrix, the culture can be freely promoted in a three-dimensional direction. In a liquid, the culture vessel wall may physically limit the direction of cell growth. Therefore, culture in an extracellular matrix is preferable as a culture condition for the three-dimensional culture of the present disclosure. In the present disclosure, the three-dimensional culture is usually performed *in vitro.*

In a preferred embodiment of the present disclosure, the three-dimensional culture can be initiated after removing the anticancer agent that was used to treat the cancer tissue or the tissue analogous to the cancer tissue. The anticancer agent can be removed by washing the tissue treated with the anticancer agent. Thereafter, the three-dimensional culture is preferably continued in the absence of the anticancer agent. To remove the anticancer agent, the tissue may be washed with a fresh basal medium such as physiological saline or DMEM/F 12.

Tissue from which the anticancer agent has been removed by washing can be mixed with a new extracellular matrix that does not contain the anticancer agent. Then, by placing a new medium (a first medium) containing no anticancer agent on the mixture of the tissue and the extracellular matrix, conditions favorable for a three-dimensional culture of the tissue can be provided. Alternatively, the cancer tissue can be placed on the extracellular matrix and a new extracellular matrix can be placed on it. Furthermore, three-dimensional culture can also be carried out by inserting the cancer tissue into the extracellular matrix.

For example, when treating a tissue (an organoid) cultured from PLR123, a cancer stem cell line derived from colorectal cancer, with an active molecule (SN-38) produced by *in vivo* decomposition of irinotecan (CPT-11), which is a general therapeutic agent for colorectal cancer, 0.1 to 10 µM can be added to the medium and treatment can be carried out for about one to five days. The preferable treatment time is, for example, about three days (72 hours). When the treated organoid is washed and three-dimensionally cultured again under conditions that allow proliferation, a cell cluster that is resistant to treatment with the anticancer agent is formed.

### Three-dimensional culture medium

The medium (first medium) that can be used for the three-dimensional culture of the present disclosure is not particularly limited as long as it can be used for three-dimensional culture of cells and tissues, and, for example, a conventionally-known basal medium or a basal medium supplemented with an additive can be used.

The known basal culture solution is not particularly limited as long as it is suitable for culturing cancer cells that are the source of cancer stem cells, and examples include DMEM/F12, DMEM, F10, F12, IMDM, EMEM, RPMI-1640, MEM, BME, Mocoy's 5A, and MCDB131.

As additives, growth factors such as EGF, bFGF, hLIF, HGF, NGF, NSF-1, TGFβ, TNFα and heparin, antibiotics, buffers such as sodium bicarbonate and HEPES, glucose, glutamine, BSA, insulin, transferrin, putrescine, selenite, progesterone, hydrocortisone, N-acetylcysteine, and combined supplements such as N-2 supplement and B-27 supplement can be used.

The concentration of EGF is not particularly limited, but is 0.1 to 100 ng/mL, preferably 0.5 to 50 ng/mL, and more preferably 1 to 20 ng/mL. The concentration of bFGF is not particularly limited, but is 0.1 to 100 ng/mL, preferably 0.5 to 50 ng/mL, and more preferably 1 to 20 ng/mL. The concentration of hLIF is not particularly limited, but is 0.1 to 100 ng/mL, preferably 0.5 to 50 ng/mL, and more preferably 1 to 20 ng/mL. The concentration of HGF is not particularly limited, but is 0.1 to 100 ng/mL, preferably 1 to 50 ng/mL. The concentration of NGF is not particularly limited, but is 0.1 to 100 ng/mL, preferably 1 to 50 ng/mL. The concentration of NSF-1 is not particularly limited, but is 0.1 to 100 ng/mL, preferably 1 to 50 ng/mL. The concentration of TGFβ is not particularly limited, but is 0.1 to 100 ng/mL, preferably 1 to 50 ng/mL. The concentration of TNFα is not particularly limited, but is 0.1 to 100 ng/mL, preferably 1 to 50 ng/mL. The concentration of heparin is not particularly limited, but is 10 ng/mL to 10 µg/mL, preferably 2 to 5 µg/mL. The concentration of BSA is not particularly limited, but is 0.1 to 10 mg/mL, preferably 1 to 8 mg/mL. The concentration of insulin is not particularly limited, but is 1 to 100 µg/mL, preferably 10 to 50 µg/mL. The concentration of transferrin is not particularly limited, but is 10 to 500 µg/mL, preferably 50 to 200 µg/mL. The concentration of putrescine is not particularly limited, but is 1 to 50 µg/mL, preferably 10 to 20 µg/mL. The concentration of selenite is not particularly limited, but is 1 to 50 nM, preferably 20 to 40 nM. The concentration of progesterone is not particularly limited, but is 1 to 50 nM, preferably 10 to 30 nM. The concentration of hydrocortisone is not particularly limited, but is 10 ng/mL to 10 µg/mL, preferably 100 ng/mL to 1 µg/mL. The concentration of D-(+)-glucose is not particularly limited, but is 1 to 20 mg/mL, preferably 2 to 10 mg/mL. The concentration of sodium bicarbonate is not particularly limited, but is 0.1 to 5 mg/mL, preferably 0.5 to 2 mg/mL. The concentration of HEPES is not particularly limited, but is 0.1 to 50 mM, preferably 1 to 20 mM. The concentration of L-glutamine is not particularly limited, but is 0.1 to 10 mM, preferably 1 to 5 mM. The concentration of N-acetylcysteine is not particularly limited, but is 1 to 200 µg/mL, preferably 10 to 100 µg/mL.

The three-dimensional culture medium (first medium) used in the methods of the present invention includes various cell culture media. A preferred cell culture medium is a defined synthetic medium which is buffered with a carbonate-based buffer to a pH of 7.4 (preferably between 7.2 and 7.6, or at least 7.2 and 7.6 or less), while the cells are cultured under an atmosphere comprising 5% to 10% CO₂, or at least 5% and 10% or less CO₂, preferably 5% CO₂.

A medium composition as described above is suitable for culturing a cancer tissue or a tissue analogous to a cancer tissue. Growth factors can also be added to the medium if the tissue requires growth factors and the like for survival or growth. In order to three-dimensionally culture tissues in such these media, an extracellular matrix is usually used in addition to the above media. When used in addition to the above-mentioned medium, the extracellular matrix has the action of providing a scaffold to the cultured tissues and cells, supporting maintenance and proliferation of the cells, and aiding tissue formation. For example, an extracellular matrix in which various growth factors and the like are admixed to a basal membrane matrix is commercially available under a trade name such as "Matrigel".

### Extracellular matrix

The three-dimensional culture medium (first medium) of the present invention may be diffused in the extracellular matrix (ECM). In a preferred method of the invention, isolated tissue fragments or isolated epithelial stem cells are attached to the ECM. The ECM consists of various polysaccharides, water, elastin, and glycoproteins. Glycoproteins include collagen, entactin (nidogen), fibronectin, and laminin. The ECM is secreted by connective tissue cells. Different types of ECMs are known, which comprise different compositions containing different types of glycoproteins and/or different combinations of glycoproteins. The ECM can be provided by culturing ECM-producing cells such as fibroblasts in a container, then removing these cells and adding isolated tissue fragments or isolated epithelial stem cells. Examples of extracellular matrix-producing cells include chondrocytes that mainly produce collagen and proteoglycans, fibroblasts that mainly produce type IV collagen, laminin, interstitial procollagen, and fibronectin, as well as colon myofibroblasts that mainly produce collagen (type I, type III, and type V), chondroitin sulfate proteoglycans, hyaluronic acid, fibronectin, and tenascin-C. Alternatively, the ECM is commercially available. Examples of commercially available extracellular matrixes are basement membrane preparations derived from an extracellular matrix protein (Invitrogen) and Engelbreth-Holm-Swarm (EHS) mouse sarcoma cells (e.g., Matrigel^{®} (BD Biosciences)). Synthetic extracellular matrix materials such as ProNectin (Sigma Z378666) may also be used. If desired, a mixture of extracellular matrix materials may also be used. The use of an ECM to culture stem cells enhances the long-term survival of stem cells and the continued presence of undifferentiated stem cells. In the presence of an ECM, three-dimensional tissue organoids that cannot be cultured in the absence of an ECM can be cultured. The extracellular matrix material is usually submerged in the bottom of the dish in which the cells are suspended. Typically, when the matrix solidifies at 37°C, the medium is added, which diffuses into the ECM. Cells in the medium adhere to the ECM by interacting with the surface structure of the ECM, for example by interacting with integrins. To coat the cell culture vessel, a fibronectin solution of about 1 mg/ml (stock solution) used at about 1 µg/cm², or about 1 µg/cm² to about 250 µg/cm², or about 1 µg/cm² to about 150 µg/cm² may be used. In some embodiments, the cell culture vessel is coated with 8 µg/cm² to 125 µg/cm² of fibronectin.

An example of an ECM for use in a culture method of the present disclosure contains at least one glycoprotein such as laminin.

Preferred ECMs for use in the culture methods of the present disclosure include at least two distinct glycoproteins, such as two different types of collagen, or collagen and laminin. The ECM may be a synthetic hydrogel extracellular matrix or a natural ECM. A more preferred ECM is provided by Matrigel^{®} (BD Biosciences). Matrigel^{®} (BD Biosciences) contains laminin, entactin, and collagen IV. In some embodiments, the extracellular matrix is a laminin-containing extracellular matrix such as Matrigel^{®} (BD Biosciences).

In some embodiments, a single stem cell, a cell population, or a tissue fragment is implanted in Matrigel^{®} (BD Biosciences). Matrigel^{®} (BD Biosciences) is optionally low in growth factors and/or phenol red-free.

In some embodiments, the culture medium is placed on top of the ECM. The culture medium can then be removed and replenished as and when needed. In some embodiments, the culture medium is replenished daily, every two days, every three days, every four days, every five days, every six days, or every seven days. If a component is "added" or "removed" from the medium, in some embodiments, it means that the medium itself is removed from the ECM, and then, a new medium containing the component to be "added", or a new medium from which the component to be "removed" has been excluded, is placed on the ECM.

In some embodiments, the culture medium used in the present disclosure is in contact with the extracellular matrix, or with a 3D matrix that mimics the extracellular matrix by interacting with a cell membrane protein, such as an integrin.

### Culture conditions

In the method for culturing a cancer tissue or a tissue analogous to a cancer tissue of the present disclosure, the following example can be given as a particularly desirable culture condition:
Tissue embedded in 50% Matrigel,
DMEM/F12 (containing HEPES and glutamine)
Penicillin/streptomycin
N2 supplement (containing insulin)
B27 supplement (containing insulin)
N-Acetylcysteine
37°C
5% CO₂
20% O₂

A culture period of about two to ten days is required to three-dimensionally culture a cancer tissue, or a tissue analogous to a cancer tissue, under general culture conditions and induce a treatment-resistant cell cluster. Whether a treatment-resistant cell cluster is formed can be known from indicators such as changes in the morphological characteristics of the cell cluster and the formation of differentiated cells. For example, when an organoid cultured from the stem cell line PLR123 derived from colon cancer is treated with an anticancer agent and further three-dimensionally cultured by a culture method of the present disclosure, a treatment-resistant cell cluster having the characteristics of an anticancer agent-resistant colon cancer tissue and containing differentiated cells is formed after culturing for two to ten days under the above culture conditions.

### Cancer tissue

In the culture method of the present disclosure, a cancer tissue generated in a cancer lesion can be cultured after being treated with an anticancer agent. For example, a cancer tissue in a surgically-resected cancer lesion, or a structure containing cancer stem cells formed in the cancer lesion, can also be cultured using a culture method of the present disclosure. The formation of a treatment-resistant cell cluster having characteristics peculiar to a patient can be expected by utilizing a cancer tissue obtained from the patient.

### Tissue analogous to cancer tissue

A cancer stem cell-derived tissue can be used as the tissue analogous to a cancer tissue disclosed of the present disclosure. A cancer stem cell-derived tissue is a multicellular cell cluster formed as proliferation and differentiation of cancer stem cells proceed. By adjusting the culture conditions, usually, cancer stem cell-derived tissues having common histochemical characteristics are formed from the same cancer stem cells, or cancer stem cell-derived tissues containing cells that differentiated and have different characteristics are formed from the same cancer stem cells.

In the present disclosure, a tissue analogous to a cancer tissue or a cancer stem cell-derived tissue can be prepared, for example, by culturing a cancer stem cell or a cell population containing a cancer stem cell. Formation of a tissue analogous to a cancer tissue or a cancer stem cell-derived tissue can be verified through morphological characteristics of the tissue and the expression states of genes that serve as a marker for stem cells or differentiated cells, after culturing for a predetermined period of time.

As a cell population containing cancer stem cells, cells into which a gene of a cancer virus such as SV40 or an oncogene such as Ras has been introduced, cells established from a cancer tissue, or the like can be used.

As a cell population containing cancer stem cells, it is preferable to use a cell population that reproduces the hierarchical structure of the cancer tissue, and for example, collected cancer tissue can be used; however, it is preferably an established cancer cell line prepared by transplanting a cancer into a non-human animal and passaging, more preferably an established cancer cell line prepared by transplanting a cancer into an immunodeficient animal and passaging, and most preferably, an NOG-established cancer cell line prepared by transplanting a cancer tissue into an NOG mouse and passaging can be used.

Furthermore, the cell population containing cancer stem cells may be a spheroid formed by spheroid culture, or a cell population containing cells that are positive for at least one or more markers selected from the cancer stem cell markers CD24, CD29, CD34, CD44, CD49f, CD56, CD90, CD117, CD133, CD135, CD166, CD184, CD271, CD326, Aldefluor, ABCG2, ABCG5, LGR5, and Msi1.

The origin of the cell population is not particularly limited, and those derived from mammals such as humans, monkeys, chimpanzees, dogs, cows, pigs, rabbits, rats, and mice can be used, but those derived from humans are preferred.

An NOG-established cancer cell line can be produced by a method known to those skilled in the art. For example, the method described in Fujii E. et al., Pathol int. 2008; 58: 559-567 can be used. It can be established by physically mincing a surgically-excised human colorectal cancer, gastric cancer, lung cancer, breast cancer, pancreatic cancer, and such with scissors, and subcutaneously transplanting into NOG mice and passaging. In NOG-established cancer cell lines, the characteristics of the original human cancer tissue are retained even after passaging.

The disclosed tissue analogous to a cancer tissue may be a cell population, or one or more organoids comprising the above-mentioned cancer stem cells, which may be prepared or obtained by a step of three-dimensionally culturing cancer stem cells or a fragment of a tissue mainly composed of cancer stem cells. The cancer stem cells, or a fragment of a tissue mainly composed of cancer stem cells, may be cultured for at least 1 day, preferably at least 2 days, 3 days, 4 days, 5 days, 6 days, 7 days, 8 days, 9 days, 10 days or longer.

The disclosed tissue analogous to a cancer tissuemay be a cell population, or one or more spheroids comprising the above-mentioned cancer stem cells, which may be prepared or obtained by a step of suspension culturing cancer stem cells or a fragment of a tissue mainly composed of cancer stem cells. The cancer stem cells, or a fragment of a tissue mainly composed of cancer stem cells, may be cultured for, at least 1 day, preferably at least 2 days, 3 days, 4 days, 5 days, 6 days, 7 days, 8 days, 9 days, 10 days or longer.

A tissue analogous to a cancer tissue formed *ex vivo* includes organoids and spheroids in particular. The culture performed to obtain a tissue analogous to a cancer tissue or a cancer stem cell-derived tissue *ex vivo* is particularly referred to as a pre-culture step.

### Pre-culture

The medium (second medium) used when culturing in a pre-culture step a tissue analogous to a cancer tissue to be cultured by a culturing method of the present disclosure is not particularly limited as long as it is a medium that can be used to culture cells or tissues. For example, a conventionally-known basal medium or a medium obtained by adding an additive to a basal medium can be used. Depending on the pre-culture mode, the second medium can be selected from media suitable for three-dimensional culture and media not suitable for three-dimensional culture. When the pre-culture is a three-dimensional culture, the second medium may be a medium having the same composition as the first medium.

The known basal culture solution is not particularly limited as long as it is suitable for culturing cancer cells that are the source of cancer stem cells, and examples include DMEM/F12, DMEM, F10, F12, IMDM, EMEM, RPMI-1640, MEM, BME, Mocoy's 5A, and MCDB131.

As additives, growth factors such as EGF, bFGF, hLIF, HGF, NGF, NSF-1, TGFβ, TNFα and Heparin, antibiotics, buffers such as sodium bicarbonate and HEPES, glucose, glutamine, BSA, insulin, transferrin, putrescine, selenite, progesterone, hydrocortisone, N-acetylcysteine, and combined supplements such as N-2 Supplement and B-27 Supplement can be used.

The concentration of EGF is not particularly limited, but is 0.1 to 100 ng/mL, preferably 0.5 to 50 ng/mL, and more preferably 1 to 20 ng/mL. The concentration of bFGF is not particularly limited, but is 0.1 to 100 ng/mL, preferably 0.5 to 50 ng/mL, and more preferably 1 to 20 ng/mL. The concentration of hLIF is not particularly limited, but is 0.1 to 100 ng/mL, preferably 0.5 to 50 ng/mL, and more preferably 1 to 20 ng/mL. The concentration of HGF is not particularly limited, but is 0.1 to 100 ng/mL, preferably 1 to 50 ng/mL. The concentration of NGF is not particularly limited, but is 0.1 to 100 ng/mL, preferably 1 to 50 ng/mL. The concentration of NSF-1 is not particularly limited, but is 0.1 to 100 ng/mL, preferably 1 to 50 ng/mL. The concentration of TGFβ is not particularly limited, but is 0.1 to 100 ng/mL, preferably 1 to 50 ng/mL. The concentration of TNFα is not particularly limited, but is 0.1 to 100 ng/mL, preferably 1 to 50 ng/mL. The concentration of heparin is not particularly limited, but is 10 ng/mL to 10 µg/mL, preferably 2 to 5 µg/mL. The concentration of BSA is not particularly limited, but is 0.1 to 10 mg/mL, preferably 1 to 8 mg/mL. The concentration of insulin is not particularly limited, but is 1 to 100 µg/mL, preferably 10 to 50 µg/mL. The concentration of transferrin is not particularly limited, but is 10 to 500 µg/mL, preferably 50 to 200 µg/mL. The concentration of putrescine is not particularly limited, but is 1 to 50 µg/mL, preferably 10 to 20 µg/mL. The concentration of selenite is not particularly limited, but is 1 to 50 nM, preferably 20 to 40 nM. The concentration of progesterone is not particularly limited, but is 1 to 50 nM, preferably 10 to 30 nM. The concentration of hydrocortisone is not particularly limited, but is 10 ng/mL to 10 µg/mL, preferably 100 ng/mL to 1 µg/mL. The concentration of D-(+)-glucose is not particularly limited, but is 1 to 20 mg/mL, preferably 2 to 10 mg/mL. The concentration of sodium bicarbonate is not particularly limited, but is 0.1 to 5 mg/mL, preferably 0.5 to 2 mg/mL. The concentration of HEPES is not particularly limited, but is 0.1 to 50 mM, preferably 1 to 20 mM. The concentration of L-glutamine is not particularly limited, but is 0.1 to 10 mM, preferably 1 to 5 mM. The concentration of N-acetylcysteine is not particularly limited, but is 1 to 200 µg/mL, preferably 10 to 100 µg/mL.

The second medium used in the methods of the present invention includes various cell culture media. A preferred cell culture medium is a defined synthetic medium which is buffered with a carbonate-based buffer to a pH of 7.4 (preferably between 7.2 and 7.6, or at least 7.2 and 7.6 or less), while the cells are cultured under an atmosphere containing 5% to 10% CO₂, or at least 5% and 10% or less CO₂, preferably 5% CO₂.

A medium composition as described above is suitable for culturing cancer stem cells. Growth factors can also be added to the medium if the tissue requires growth factors and the like for survival or growth. In order to three-dimensionally culture tissues in these media, an extracellular matrix is usually used in addition to the above media. When used in addition to the above-mentioned medium, the extracellular matrix has the action of providing a scaffold to the cultured tissues and cells, supporting maintenance and proliferation of the cells, and aiding tissue formation. For example, an extracellular matrix in which various growth factors and the like are admixed to a basal membrane matrix is commercially available under a trade name such as "Matrigel".

When the pre-culture step is a three-dimensional culture, the second medium of the present invention may be diffused in the extracellular matrix (ECM). In a preferred method, isolated tissue fragments or isolated epithelial stem cells are attached to the ECM. The ECM consists of various polysaccharides, water, elastin, and glycoproteins. Glycoproteins include collagen, entactin (nidogen), fibronectin, and laminin. The ECM is secreted by connective tissue cells. Different types of ECMs are known, which comprise different compositions containing different types of glycoproteins and/or different combinations of glycoproteins. The ECM can be provided by culturing ECM-producing cells such as fibroblasts in a container, then removing these cells and adding isolated tissue fragments or isolated epithelial stem cells. Examples of extracellular matrix-producing cells include chondrocytes that mainly produce collagen and proteoglycans, fibroblasts that mainly produce type IV collagen, laminin, interstitial procollagen, and fibronectin, as well as colon myofibroblasts that mainly produce collagen (type I, type III, and type V), chondroitin sulfate proteoglycans, hyaluronic acid, fibronectin, and tenascin-C. Alternatively, the ECM is commercially available. Examples of commercially available extracellular matrixes are basement membrane preparations derived from an extracellular matrix protein (Invitrogen) and Engelbreth-Holm-Swarm (EHS) mouse sarcoma cells (e.g., Matrigel^{®} (BD Biosciences)). Synthetic extracellular matrix materials such as ProNectin (Sigma Z378666) may also be used. If desired, a mixture of extracellular matrix materials may also be used. The use of an ECM to culture stem cells enhances the long-term survival of stem cells and the continued presence of undifferentiated stem cells. In the presence of an ECM, three-dimensional tissue organoids that cannot be cultured in the absence of an ECM can be cultured. The extracellular matrix material is usually submerged in the bottom of the dish in which the cells are suspended. Typically, when the matrix solidifies at 37°C, the medium is added, which diffuses into the ECM. Cells in the medium adhere to the ECM by interacting with the surface structure of the ECM, for example by interacting with integrins. To coat the cell culture vessel, a fibronectin solution of about 1 mg/ml (stock solution) used at about 1 µg/cm², or about 1 µg/cm² to about 250 µg/cm², or about 1 µg/cm² to about 150 µg/cm² may be used. In some embodiments, the cell culture vessel is coated with 8 µg/cm² to 125 µg/cm² of fibronectin.

An example of an ECM for use in a culture method of the present disclosure contains at least one glycoprotein such as laminin.

Preferred ECMs for use in a preculture of the present disclosure include at least two distinct glycoproteins, such as two different types of collagen or collagen and laminin. The ECM may be a synthetic hydrogel extracellular matrix or a natural ECM. A more preferred ECM is provided by Matrigel^{®} (BD Biosciences). Matrigel^{®} (BD Biosciences) contains laminin, entactin, and collagen IV. In some embodiments, the extracellular matrix is a laminin-containing extracellular matrix such as Matrigel^{®} (BD Biosciences).

A single stem cell, a cell population, or a tissue fragment may be implanted in Matrigel^{®} (BD Biosciences). Matrigel^{®} (BD Biosciences) is optionally low in growth factors and/or phenol red-free.

In some embodiments, the culture medium is placed on top of the ECM. The culture medium can then be removed and replenished as and when needed. In some embodiments, the culture medium is replenished daily, every two days, every three days, every four days, every five days, every six days, or every seven days. If a component is "added" or "removed" from the medium, in some embodiments, it means that the medium itself is removed from the ECM, and then, a new medium containing the component to be "added", or a new medium from which the component to be "removed" has been excluded, is placed on the ECM.

The second culture used in the present disclosure may be in contact with the extracellular matrix, or with a 3D matrix that mimics the extracellular matrix by interacting with a cell membrane protein, such as an integrin.

When the pre-culture of the present disclosure is a three-dimensional culture of cancer stem cells, the following example can be given as a particularly desirable culture condition:
Tissue embedded in 50% Matrigel,
DMEM/F12 (containing HEPES and glutamine)
Penicillin/streptomycin
N2 supplement (containing insulin)
B27 supplement (containing insulin)
N-Acetylcysteine
37 °C
5% CO₂
20% O₂

### Stem cells

Stem cells are found in many organs of adult humans and mice. Although the exact characteristics of adult stem cells in individual tissues may vary widely, adult stem cells share at least the following characteristics: they retain an undifferentiated phenotype; progenies can differentiate into all lineages present in the relevant tissue; they retain self-sustaining ability throughout life; and they can regenerate related tissues following an injury. Stem cells are located in special locations called stem cell niches. Stem cell niches provide appropriate intercellular adhesions and signals to maintain the stem cell populations. The stem cells disclosed in the present disclosure preferably express LGR5.

### Cancer stem cells

The origin of the cancer stem cells of the present disclosure is not particularly limited, but is preferably a human tumor tissue or a human cell line.

In the present invention, "cancer" typically refers to a physiological condition in mammals characterized by uncontrolled cell proliferation, or to a related physiological condition. The types of cancer in the present invention include, but are not limited to, carcinomas (epithelial cancers), such as pancreatic cancer, prostate cancer, breast cancer, skin cancer, gastrointestinal cancer, lung cancer, hepatocellular carcinoma, cervical cancer, endometrial cancer, ovarian cancer, fallopian tube cancer, vaginal cancer, liver cancer, bile duct cancer, bladder cancer, urinary tract cancer, thyroid cancer, adrenal cancer, kidney cancer, and other glandular tissue cancers. Sarcomas (non-epithelial carcinomas) include liposarcoma, leiomyosarcoma, rhabdomyosarcoma, synovial sarcoma, angiosarcoma, fibrosarcoma, malignant peripheral nerve tumor, gastrointestinal stromal tumor, desmoid tumor, Ewing's sarcoma, osteosarcoma, chondrosarcoma, leukemia, lymphoma , myeloma, and other solid organ tumors, e.g., melanoma or brain tumor (Kumar V, Abbas AK, Fausio N. Robbins and Cotran Pathologic Basis of Disease. 7th Ed. Unit I: General Pathology, 7: Neoplasia, Biology of tumor growth: Benign and malignant neoplasms. 269-342, 2005). The cancer stem cells of the present invention may be derived from tumor tissues derived from the above epithelial cancers. Alternatively, they can be a cell line established from a cancer stem cell.

In the present disclosure, "cancer stem cells" refer to cells having the abilities of i) and/or ii) below.
i) Self-replicating ability. Self-replicating ability refers to the ability of one or both of two divided daughter cells to produce cells that retain abilities and degree of differentiation equivalent to those of the parent cells according to the cell lineage.
ii) Ability of differentiating into multiple types of cancer cells that constitute a cancer cell mass. As with normal stem cells, multiple types of cancer cells differentiated from a cancer stem cell form a hierarchical structure in which the cancer stem cell is at the apex according to the cell lineage. The stepwise production of various types of cancer cells from a cancer stem cell leads to formation of a cancer cell mass having various properties.

Cancer stem cells are cancer cells that have cancer-forming ability and possess pluripotency and self-replicating ability like normal stem cells. In addition, they may be positive for at least one or more cancer stem cell markers selected from CD24, CD29, CD34, CD44, CD49f, CD56, CD90, CD117, CD133, CD135, CD166, CD184, CD271, CD326, Aldefluor, ABCG2, ABCG5, LGR5, and Msi1, but are not necessarily required to be positive for these markers. Preferably, at least one or more markers selected from CD326, CD133, CD44, ALDH, and LGR5 are positive. More preferably, at least two or more markers selected from CD326, CD133, CD44, ALDH, and LGR5 are positive. Even more preferably, at least three or more markers selected from CD326, CD133, CD44, ALDH, and LGR5 are positive. Most preferably, all of CD326, CD133, CD44, ALDH, and LGR5 are positive.

In the present disclosure, cancer stem cells can be stem cells obtained from cancer tissues, or additionally, cell lines established from those stem cells. PLR59 and PLR123 (STEM CELLS 2012; 30: 2631-2644), which are stem cell lines derived from human colorectal cancer, are representative cancer stem cell lines. When a cell line is used as a starting material, target cell clusters can be obtained in a highly reproducible manner and in a homogeneous state.

### How to form a cell cluster

The present disclosure provides a method for forming a cell cluster that is treatment-resistant to a therapeutic agent for diseases. The method comprises the step of three-dimensionally culturing a cancer tissue or a tissue analogous to a cancer tissue which was treated with an anticancer agent. Each step of the method of the present disclosure can be carried out according to a method detailed above as a method for culturing a cancer tissue, or a tissue analogous to a cancer tissue. That is, the present disclosure relates to a method for producing a cell cluster that is resistant to treatment with an anticancer agent, which comprises the step of three-dimensionally culturing a cancer tissue or a tissue analogous to a cancer tissue which was treated with an anticancer agent. In the present disclosure, the cell cluster that is ultimately formed is formed from cells that survived treatment with the anticancer agent. Therefore, it is a tissue having resistance to the anticancer agent used for the treatment. The method of the present disclosure for producing a cell cluster that is resistant to treatment with an anticancer agent comprises a step of isolating, after three-dimensional culture of a cancer tissue, a cell cluster that is resistant to treatment with an anticancer agent induced by the three-dimensional culture from the culture system.

In the present disclosure, a "cell cluster" includes organoids formed by three-dimensionally culturing a tissue after treatment with an anticancer agent.

### Treatment-resistant cell cluster

Specifically, the cell cluster having resistance to the anticancer agent (treatment-resistant cell cluster) formed by culturing a cancer tissue, or a tissue analogous to a cancer tissue, in this manner has at least one of the following histochemical characteristics:
- it shows protrusions on the tissue surface composed of monolayer to multilayer cuboidal cells;
- it is composed of large circular-oval cells containing a large amount of cell debris in the cell cluster or lumen;
- compartmentalization of the tumor cell cluster is observed;
- from those compartmentalized structures, new glandular structures are subsequently formed;
- the expression of LGR5 (Uniprot ID: O75473, Refseq accession No. NP_003658) temporarily decreases, but then recovers; and
- CD44 (Uniprot ID: P16070) which is also a stem cell marker, especially CD44v9 (also called CD44v8-10, CD44E, Uniprot ID: P16070-10, Refseq accession No. NP_001001390.1, NM_001001390.1), is also positive.

The above histochemical characteristics are in good agreement with small cluster structures found in tissues of colorectal cancer patients administered with chemotherapeutic agents.

The present disclosure also relates to cell clusters formed by a method of the present invention, which are resistant to treatment with anticancer agents. The cell clusters of the present disclosure have resistance to the anticancer agents utilized in their formation. In a preferred embodiment, the cell clusters of the present disclosure express cancer stem cell markers. The cell clusters of the present disclosure also express the cancer stem cell markers LGR5 and CD44v9 in a preferred embodiment. In yet another embodiment, the cell clusters of the present disclosure also express glutathione-metabolizing enzymes such as GPX2. In a further embodiment, the cell clusters of the present disclosure are negative for the expression of Vimentin (Uniprot ID: P08670, Refseq accession No. NP_003371) and ZEB1 (Uniprot ID: P37275, Refseq accession No. NP_110378). In a more preferred embodiment, the cell clusters of the present disclosure are weakly positive for the epithelial marker E-cadherin (E-cadherin, Uniprot ID: P12830, Refseq accession No. NP_004351). Alternatively, the cell clusters of the present disclosure are histologically composed of 10 to 30 cells and typically have a size of 100 µm or less. In a preferred embodiment, the cell clusters have a glandular structure as the cells differentiate.

These characteristics mean that they are in good agreement with the characteristics of small clusters found in tissues of colorectal cancer patients who have actually undergone chemotherapy. Therefore, by observing the cell clusters of the present disclosure, it is possible to know the characteristics of tissues that have acquired anticancer agent resistance, which occur in cancer tissues during anticancer agent treatment.

### Use of the cell clusters

In other words, the present disclosure also relates to methods of analyzing states such as gene expression profile, gene expression level, protein expression profile, protein expression level, protein modification, protein localization, epigenome, and metabolomics, using cells constituting a cell cluster formed by the present disclosure as target. The cell cluster formed by the present disclosure is a model having the characteristics of an anticancer agent-resistant tissue generated in a cancer tissue that has undergone chemotherapy. Therefore, by observing its properties and comparing it, for example, with the characteristics of anticancer agent-responsive cancer tissues, changes specific to tissues that have acquired anticancer agent resistance can be identified. Alternatively, by tracking changes in these properties in the process of forming a cell cluster of the present disclosure, it is possible to understand the process of anticancer agent-resistant tissue formation.

### Gene expression/profile analysis using a cell cluster

In the present disclosure, DNA microarrays are useful for analyzing gene expression profiles and gene expression levels. By using a microarray that covers most of the human genes, the state of intracellular gene expression can be comprehensively analyzed. When the analysis target is mRNA, a cDNA microarray is used. Alternatively, a microarray equipped with a probe for miRNA is used for miRNA analysis. A gene in which an expression pattern (information or downregulation) specific to a tissue that has acquired anticancer agent resistance is observed as compared with a control as a result of expression analysis using a microarray can be identified as a gene characteristic of anticancer agent resistance acquisition. In this case, for example, a tissue sensitive to the same anticancer agent can be used as a control. In a preferred embodiment, generally, by making conditions other than the anticancer agent sensitivity as common as possible with the control, the changes specific to resistance acquisition could be easily found. Examples of conditions made in common include the type of tissue or cell, culture conditions, and the like. Alternatively, comprehensive analyses utilizing various liquid chromatographies and mass spectrometers can be used to compare protein expression profiles and protein expression levels. In addition, also known is the technique of analyzing the function of a deleted gene (loss of function) from changes in phenotype arising from the gene deletion (knockdown or knockout). For gene deletion, for example, the method of mixing shRNA and CRISPR and introducing them into cells can be used. Use of an shRNA library enables comprehensive deletion of various genes. If any cell function is lost or imparted due to a gene deletion, the role of the gene can be understood.

Focusing on a specific protein that can be found through an analysis such as the above, analysis of its modification and localization is also carried out. For example, changes in cell signaling can be clarified from changes in receptor phosphorylation and dephosphorylation, and changes in ubiquitination of an intracellular signaling factor.

### Epigenome analysis using a cell cluster

Furthermore, epigenome analysis is useful for knowing the state of regulation of gene expression in cells and tissues. Epigenome analysis can find the correlation between changes in a gene expression regulation mechanism and acquisition of anticancer agent resistance through methylation of genomic DNA and modification of histones. It is said that aspects of the stemness and differentiation of cancer cells are regulated by changes in the epigenome.

In addition, it is known that the state of energy metabolism of cells such as glycolysis and oxidative phosphorylation (OXPHOS) differs between cancer stem cells and differentiated cells. Therefore, by analyzing the metabolomics state of a cell cluster or tissue of the present disclosure, the relationship between the cells' state of energy metabolism and the mechanism of anticancer agent resistance acquisition can be clarified.

Cell clusters obtained by the present disclosure can be used in methods for searching for a target molecule of a pharmaceutical and methods for evaluating a pharmaceutical. Examples of methods for evaluating a pharmaceutical include methods of screening for a pharmaceutical and methods of screening for an anticancer agent. Pharmaceuticals include those having an action of cancelling the anticancer agent resistance acquired by the cancer cells and those that inhibit the acquisition of anticancer agent resistance.

Methods of searching for target molecules include, but are not limited to, methods for identifying genes such as DNAs and RNAs highly expressed in cancer stem cells (e.g., cancer stem cell markers) using Gene-chip analysis, and methods for identifying proteins, peptides, or metabolites highly expressed in cancer stem cells using proteomics.

Screening methods for searching for target molecules include methods in which substances that inhibit the growth of cancer stem cells are screened from a small molecule library, antibody library, micro RNA library, or RNAi library, using cell growth inhibition assay. After an inhibiting substance is obtained, its target can be revealed.

After antibodies are obtained through immunization with a cell cluster of the present disclosure or its constituent cells, binding antibodies can be screened by ELISA, or growth-inhibiting antibodies can be screened by cell growth inhibition assay. After binding antibodies or functional antibodies are obtained, their antigens can be identified to reveal target molecules.

By using a cell cluster of the present disclosure, it is possible to evaluate the effect of an existing agent or an agent under development on the cell cluster, and find a new medicinal effect.

That is, the present disclosure relates to a method for identifying, or a method of screening for, an agent having a therapeutic effect on an anticancer agent-resistant cancer using a cell cluster of the present disclosure.

The "test substances" or "test agents" in the methods of the present disclosure include, but are not particularly limited to, for example, single compounds such as natural compounds, organic compounds, inorganic compounds, proteins, antibodies, peptides, and amino acids, and compound libraries, expression products from gene libraries, cell extracts, cell culture supernatants, products from fermentative microorganisms, extracts from marine organisms, plant extracts, prokaryotic cell extracts, unicellular eukaryotic cell extracts, or animal cell extracts. These may be purified products or may be crude products such as extracts from plants, animals, microorganisms, and such. In addition, methods of producing test substances are not particularly limited, and such substances may be isolated from natural products, chemically or biochemically synthesized, or prepared by genetic engineering.

The above test samples may be suitably labeled as needed. Labels include, for example, radioactive labels and fluorescent labels. In addition to the above test samples, mixtures of a plurality of these test samples are included.

In this method, methods of administering a test substance to a non-human animal model are not particularly limited. Depending on the type of the test substance to be administered, oral administration or parenteral administration such as subcutaneous, intravenous, topical, transdermal, and enteral (rectal) administration can be appropriately selected.

In this method, methods of treating a cell cluster with a test substance are not particularly limited. The treatment can be conducted by adding a test sample to the culture solution of the cell cluster or its cell extract. When the test sample is a protein, for example, a vector containing DNA encoding the protein can be introduced into a cancer stem cell population, or the vector can be added to the cell extract of a cancer stem cell population. In addition, for example, a two-hybrid method using yeast or animal cells can be used.

Specifically, the present disclosure provides a method for identifying an agent as follows:
a method for identifying an agent having a therapeutic activity for a disease, wherein the method comprises:
(i) selecting at least one test agent;
(ii) contacting a cell cluster of the present disclosure with the test agent; and
(iii) measuring the cytotoxic activity of the agent on cells contained in the cell cluster or tissue.

In a preferred embodiment, the present disclosure further additionally comprises (iv) selecting a test agent that has greater cytotoxic activity as compared to a control.

The identification method of the present disclosure can also be carried out by the following steps:
a method for identifying an agent having a therapeutic activity for a disease, wherein the method comprises:
(i) selecting at least one test agent;
(ii) contacting a cell cluster of the present disclosure with the test agent; and
(iii) comparing the protein expression of cells contained in the cell cluster in the presence and absence of the agent.

In a preferred embodiment, the method can further additionally comprise (iv) selecting a test agent which resulted in an altered protein expression as compared to a control.

The present disclosure also relates to a method for determining cytotoxic activity including the following steps:
a method of determining the cytotoxic activity of an agent, wherein the method comprises:
(i) contacting a test agent with a cell cluster of the present disclosure.

In a preferred embodiment, the method further additionally comprises (ii) detecting the cytotoxic effect of the test agent when the cytotoxic effect is greater as compared to that of a control.

The present disclosure relates to a method for identifying the mechanism of action of an agent including the following steps:
a method for identifying the mechanism of action of an agent, wherein the method comprises:
(i) selecting at least one test agent;
(ii) contacting a cell cluster of the present disclosure with the test agent; and
(iii) comparing the protein expression of cells contained in the cell cluster in the presence and absence of the agent.

In a preferred embodiment of the present disclosure, the method can further additionally include (iv) identifying a protein as a target of the agent if the protein expression is altered as compared to a control. In the above identification method, for example, if the test agent is an agent for which cytotoxic activity against the cell cluster of the present disclosure was preliminarily found, there is a possibility that a molecule whose expression changes due to the action of the agent is a target molecule of the agent. A molecule thus identified is useful as a therapeutic target for a cancer that has acquired agent resistance or as a target molecule for inhibiting the acquisition of agent resistance.

Furthermore, the present disclosure relates to the following identification method:
a method for identifying a combination for combined use of an agent, wherein the method comprises:
(i) providing a cell cluster resistant to treatment with a first test agent;
(ii) contacting the provided cell cluster with a second test agent which is different from that of (i); and
(iii) measuring the cytotoxic activity of the second test agent against cells contained in the cell cluster of (i).

In a preferred embodiment, the above method can further additionally include (iv) determining a combination for combined use of the first test agent and a test agent which had a higher cytotoxic activity as compared with a control. In the above method, the cell cluster of step (i) can be a cell cluster of the present disclosure.

In each of the above screening methods provided by the present disclosure, the control can be appropriately selected according to the purpose. In general, it is desirable that the control be a tissue or cell whose conditions other than treatment resistance are as equal as possible. One skilled in the art can set an appropriate control according to the purpose of evaluation of each property.

The present disclosure can also be carried out by the following method:
a method for identifying a combination for combined use of an agent, wherein the method comprises:
(i) providing a cell cluster resistant to treatment to a first test agent;
(ii) contacting the provided cell cluster with a second test agent which is different from that of (i); and
(iii) comparing the protein expression of cells contained in the cell cluster in the presence and absence of the test agent of (ii).

In a preferred embodiment, the above method can further additionally include (iv) determining a combination for combined use of the first agent and the second agent when the protein expression differs between the presence and absence of the second test agent. In the above method, a cell cluster of the present disclosure can be used as the cell cluster of step (i).

In the above identification method, the disease can specifically be cancer. More specifically, the cancer can be selected from the group consisting of colorectal cancer, lung cancer, breast cancer, prostate cancer, and pancreatic cancer. Further, the "change in protein expression" compared in the above steps includes a change in the protein expression profile and the protein expression level. By knowing of a change in the expression profile of a protein, it is possible to select a candidate therapeutic target for a test agent or a combination for combined use thereof. Alternatively, changes in the expression level of a specific protein show that inhibition of the expression of a certain gene was provided by a test agent or by its combination for combined use. Furthermore, for example, if the protein is a factor involved in cell proliferation signaling, the cancer therapeutic effect of the test agent or of the combination for combined use can be known from a change in protein expression. Alternatively, if it is a factor that is predicted to be involved in cancer growth or agent resistance, its inhibition can be expected to have effects such as growth inhibition and cancellation of agent resistance. Further, if it is a protein that has been determined to be a therapeutic target by an above-mentioned identification method, a candidate for a therapeutic agent that targets the protein can be identified.

On the other hand, it is possible to evaluate the action of the test agents and their combinations for combined use identified through the above methods in inhibiting the growth of tissues from cancers actually clinically determined to be agent-resistant cancers, or in inhibiting the growth of cells deriving from them, or in cancelling the resistance.

Alternatively, when the target molecule of a test agent is identified, screening of an agent that prevents the acquisition of or cancels the anticancer agent resistance becomes possible by selecting a compound that regulates the activity of the identified target molecule.

The preventively-effective substances or therapeutically-effective substances for human cancer diseases selected by a screening method of the present disclosure described above may be further subjected to other drug efficacy tests, safety tests, and such as necessary, and further subjected to clinical trials on patients with human cancer diseases, to select highly efficacious and highly practical preventively-effective substances or therapeutically-effective substances.

The preventively-effective substances or therapeutically-effective substances selected in this manner may be industrially produced by chemical synthesis, biochemical synthesis (fermentation), or genetic manipulation based further on results of their structural analysis.

### [Examples]

### [Example 1] Preparation and evaluation of cancer stem cell organoids

### 1-1. Preparation of cancer stem cell organoids

PLR123 cells were two-dimensionally (2D) cultured according to a previous report (Stem Cells, 30, 2631-2644 (2012)) in a stem cell medium (DMEM/F12 (Cat # 11330-057), 1 × Antibiotic-Antimycotic, 1 × N-2 Supplement, 4 mg/mL AlbuMAX I Lipid-Rich BSA, 20 ng/mL human EGF, 20 µg/mL human insulin (all from Thermo Fisher Scientific), 2.9 mg/mL glucose (Merck), 4 µg/mL heparin (Merck), 10 ng/mL human FGF2 (Reprocell)) at 37°C in a 5% CO₂ environment.

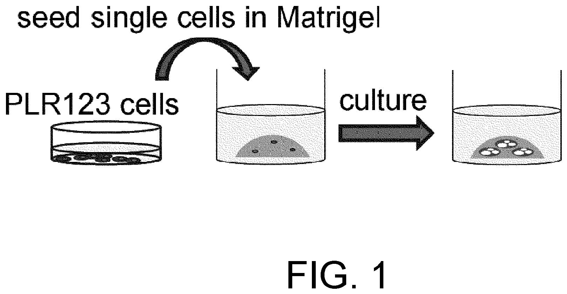

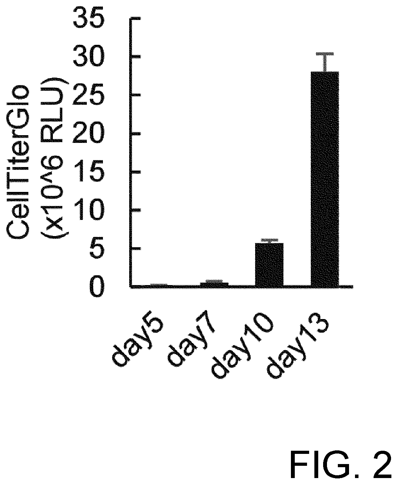

Next, the 2D-cultured cells were three-dimensionally (3D) cultured to form PLR123 organoids. Specifically, 2D-cultured cells were treated with Accutase (Innovative Cell Technologies) to prepare a single cell suspension, which is then suspended in Matrigel (Cat # 356231, Corning) and seeded on a 24-well plate at 250 cells/50 µL Matrigel droplet/well. After gelling Matrigel at 37°C, 650 µL/well of an organoid culture medium (Advanced DMEM/F12, penicillin/streptomycin, 10 mM HEPES, 2 mM Glutamax-I, 1 × B-27 Supplement, 1 × N- 2 Supplement (above Thermo Fisher Scientific), 1 mM N-acetylcysteine (Merck)) (Gastroenterology, 141, 1762-1772 (2011)) was added and 3D-culture was carried out in a 5% CO₂ environment at 37 °C to prepare organoids (Fig. 1). When the growth of organoids was measured using the CellTiter-Glo 3D Cell Viability Assay (Promega), it was confirmed that the organoids proliferated significantly from day 7 and on (n = 3, Fig. 2).

### 1-2. Gene expression analysis of cancer stem cell organoids

The gene expression of the organoids prepared in Example 1-1 was analyzed by RNA-seq. First, total RNA was prepared from 2D-cultured cells and 3D-culturted organoids (day 10) cultured in Example 1-1 using TRIzol Reagent (Cat # 15596018, Thermo Fisher Scientific). The quality of the purified total RNA was verified through RNA integrity number (RIN) measured using an Agilent 2100 Bioanalyzer (Agilent) to confirm that the RIN was greater than 9. Next, cDNAs were synthesized and amplified from 1 ng of total RNA using the SMART-Seq v4 Ultra Low Input RNA Kit for Sequencing (Takara Bio). Subsequently, a library was prepared using the Nextera XT DNA Library Prep kit (Illumina), and sequenced at 75 bp paired-end reads using the NextSeq500 system (Illumina). The obtained sequence information was mapped to NCBI RefSeq transcript sequence using RSEM software (v1.2.31), and the gene expression level was calculated by FPKM (Fragments Per Kilobase of exon model per Million mapped reads).

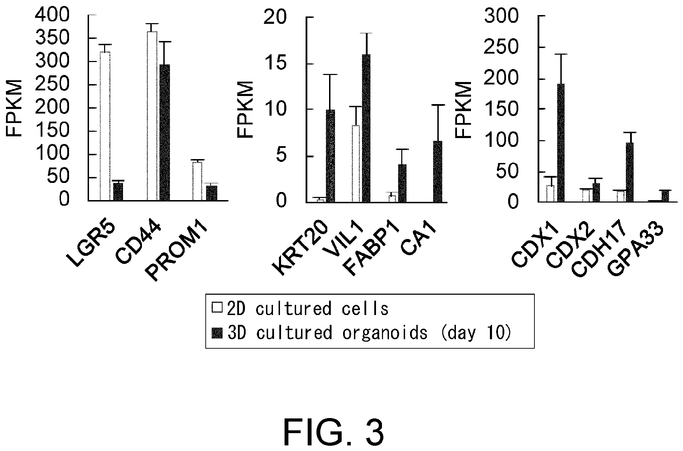

Analysis showed that the expression of the stem cell markers LGR5, CD44, and PROM1 was decreased, and the expression of differentiation markers KRT20, VIL1, FABP1, CA1, CDX1, CDX2, CDH17, and GPA33 was increased in the 3D-cultured organoids (day10), inferring that the cells had differentiated (n = 3, Fig. 3).

### 1-3. Structural analysis and LGR5 expression analysis of cancer stem cell organoids

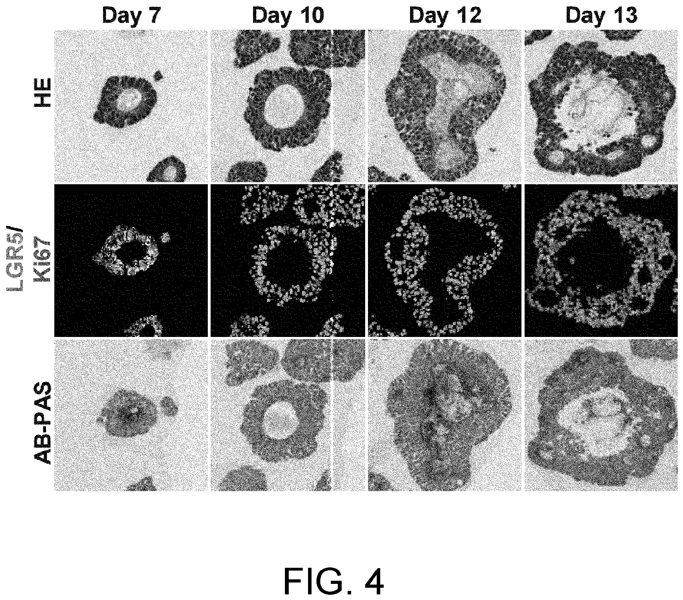

In order to observe the structure of the organoids produced in Example 1-1 in detail, a pathological analysis was performed according to a previous report (J. Toxicol. Pathol. 31, 81-85 (2018)). Specifically, after removing the culture supernatant from each of the three-dimensional cultures (day 7, day10, day 12, and day13), 4% paraformaldehyde/phosphate buffer (Cat # 09154-14, Nacalai Tesque) was added, and fixation was carried out at room temperature for 30 minutes. Matrigel was decomposed by pipetting, and organoids were collected in a centrifuge tube and washed. Subsequently, the organoids were suspended in a warmed 2% aqueous agarose solution (agarose, Type IX, Cat # A5030, Merck), centrifuged, and then cooled on ice to gel the agarose. This was treated by the AMeX method and embedded in paraffin. Paraffin blocks were sliced and stained with hematoxylin and eosin (HE) or Alcian blue-Periodic acid-Schiff (AB-PAS) by standard methods. Further, anti-LGR5 antibody (clone 2U2E-2 (Stem Cells, 30, 2631-2644 (2012))) and anti-Ki-67 antibody (clone MIB-1, Cat # M7240, Agilent) were used for immunofluorescent staining of the paraffin block sections. For LGR5, a polymer-horseradish peroxidase-labeled secondary antibody (Cat # K4001, Agilent) and Alexa Fluor 488-labeled tyramide (Cat # T20912, Thermo Fisher Scientific) were used (Acta Histochemica et Cytochemica, 48, 159-164 (2015)). As a result, a luminal structure and mucus retention were confirmed inside the organoids, and the expression of the stem cell marker LGR5 was decreased in the organoids on and after day 10 of the three-dimensional culture (Fig. 4). From the above, it was shown that PLR123 organoids reproduce traits peculiar to differentiated colorectal cancer and are a model that well reflect clinical colorectal cancer.

### [Example 2] Preparation and evaluation of regrowing cancer stem cell organoids

### 2-1. Preparation of regrowing cancer stem cell organoids

In order to analyze the mechanism of resistance to chemotherapeutic agents in cancer, cancer stem cell organoids were treated with an anticancer agent and then re-cultured to prepare regrowing organoids.

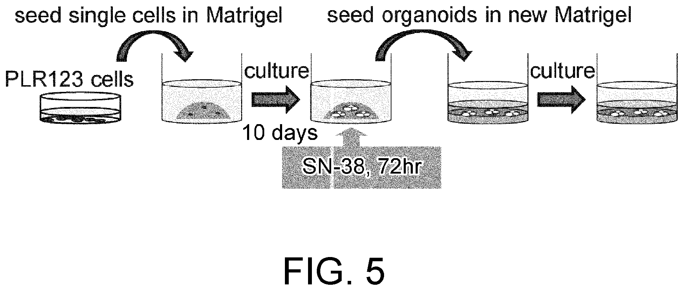

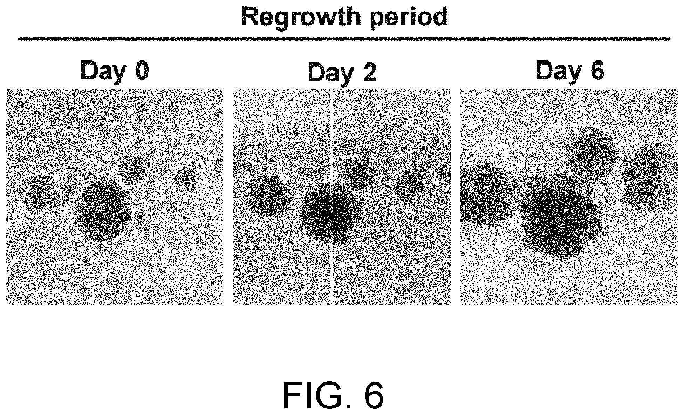

Specifically, 7-ethyl-10-hydroxycamptothecin (SN-38, Cat # H0165, Merck) was added to the organoids (day 10) prepared in Example 1-1 at a concentration of 300 nM and culture was carried out for three days (SN-38 treatment). After culturing, organoids were recovered by decomposing Matrigel with Dispase I (Cat # 386-02271, Fujifilm Wako Pure Chemical Corporation), washed with Cellotion (Cat # CB051, Nippon Zenyaku Kogyo Co., Ltd.), and the washed organoids were suspended in a new Matrigel and dispensed at 20 µL/well into a 96-well plate in which 20-30 µL/well of Matrigel was gelled beforehand. By centrifuging immediately after dispensing, the organoids were aligned on a single plane, making it easier to observe under a microscope. After gelling Matrigel at 37°C, 200 µL/well of an organoid culture medium (Example 1-1) was added and re-culture was carried out at 37°C in a 5% CO₂ environment. Microscopic observation conducted over time showed fine ridges on the surface of the organoids on day 2 of re-culture, and growth of the organoids was observed on and after day 6 of re-culture (Figs. 5 and 6).

### 2-2. Structural analysis and LGR5 expression analysis of regrowing cancer stem cell organoids

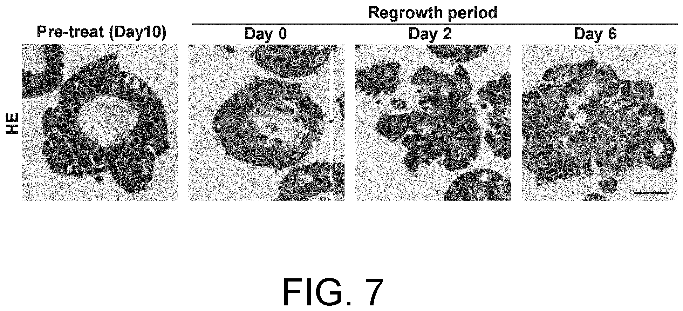

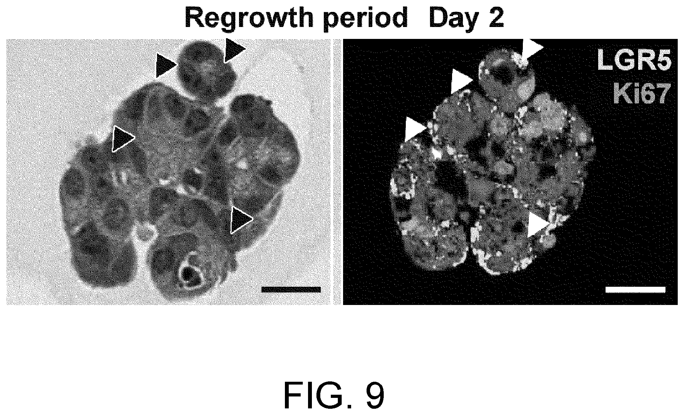

The structure of the regrowing organoids was analyzed by pathological analysis in the same manner as in Example 1-3. The analysis showed that the organoid structure was once disrupted by the SN-38 treatment after the SN-38 treatment, but a new luminal structure was formed starting from the surviving cells during re-culture (Fig. 7). In particular, on day 2 of re-culture, many small cell clusters containing LGR5-positive cells were observed (Fig. 9).

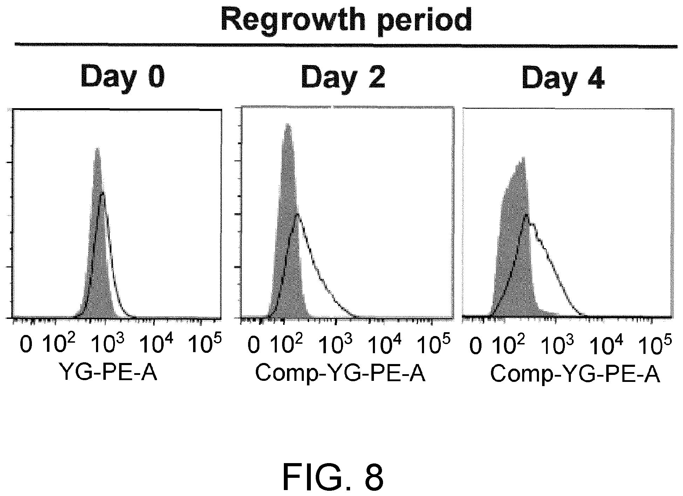

LGR5 expression of regrown organoids was measured by FACS. An SN-38-treated organoid resuspension was dispensed into a 24-well plate at 50 µL Matrigel droplets/well and re-cultured to prepare regrown organoids for FACS. The regrown organoids were recovered by Dispase I and further treated with Accutase to prepare a single cell suspension. The resulting cells were reacted with 2 µg/mL anti-LGR5 antibody (clone 2L36 (Stem Cells, 30, 2631-2644 (2012))) or control mouse IgG2a antibody (clone MG2a-53, Cat # 401502, BioLegend) at 4°C for 1 hour, and then reacted with a PE-labeled secondary antibody (Cat # P-21139, Thermo Fisher Scientific) for 30 minutes at 4°C. Then dead cells were stained with 7-AAD Viability Dye (Cat # A07704, Beckman Coulter) and measured with a flow cytometer (BD FACSAria III, BD Biosciences). PBS containing 2% fetal bovine serum and 0.1% NaN₃ was used as the buffer. Data were analyzed using BD FACSDiva (version 8.0.1, BD Biosciences) and FlowJo (version 7.6.5, BD Biosciences). While almost no LGR5 expression was observed in the organoids immediately after SN-38 treatment, it was confirmed that a part of the cells in the regrowing organoids became LGR5-positive, supporting the results of the pathological analysis (Fig. 8).

### [Example 3] Analysis of the contribution of GPX1/GPX2 towards regrowing cancer stem cell organoids

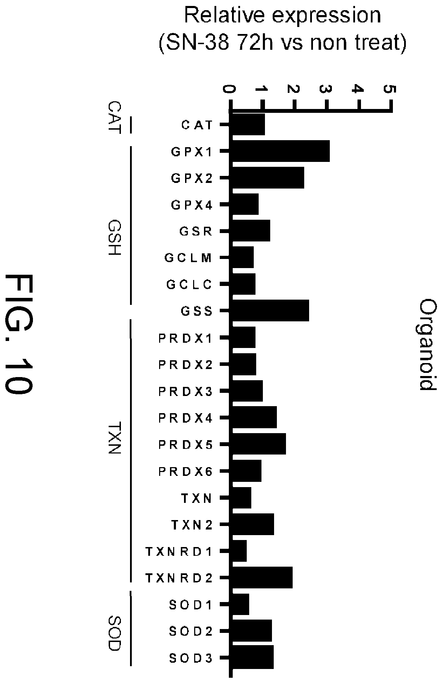

Gene expression analysis was performed on the organoids of Example 1-1 (day 10) and the SN-38-treated organoids of Example 2-1 using RNA-seq in the same manner as in Example 1-2 (n = 3). Focusing on ROS metabolism-related genes, it was shown that the expression of GPX1 and GPX2 in the glutathione antioxidant pathway had increased by two-fold or more by the SN-38 treatment (Fig. 10).

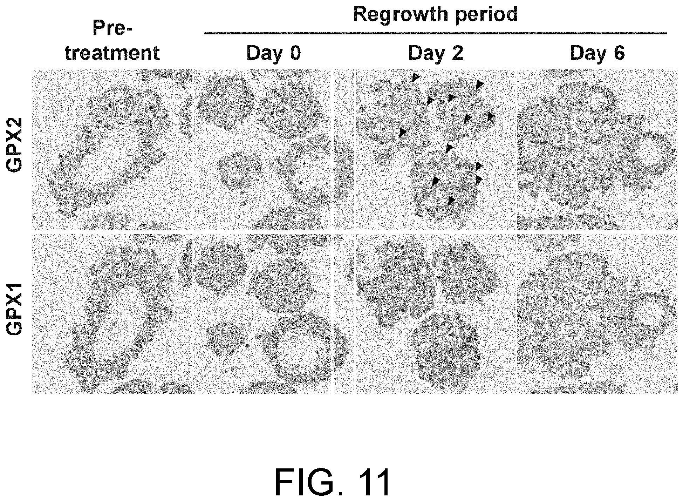

The protein expression of GPX1 (Uniprot ID: P07203, Refseq accession No. NP_000572) and GPX2 (Uniprot ID: P18283, Refseq accession No. NP_002074) was analyzed by IHC. Using an anti-GPX1 antibody (Cat # ab22604, Abcam, 0.5 µg/mL) and an anti-GPX2 antibody (Cat # ab13743 1, Abcam, 0.5 µg/mL) with the Envision system (Cat # K4003, Agilent) and peroxidase-diaminobenzidine reaction, the organoids of Example 1-1 (day 10), the SN-38-treated organoids of Example 2-1, and the regrowing organoids of Example 2-1 (day 2, day 6) were each stained. The results of IHC analysis confirmed that the expression of GPX1 and GPX2 increased immediately after the treatment with SN-38 and expression continued even on day 2 of regrowth (Fig. 11).

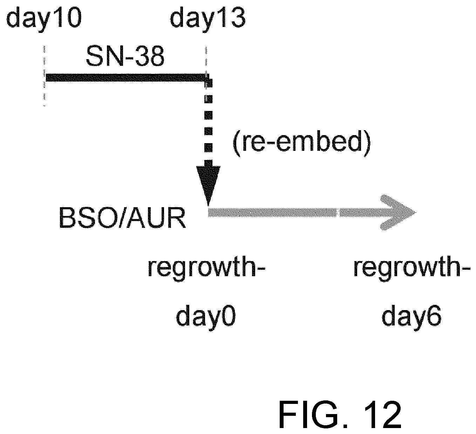

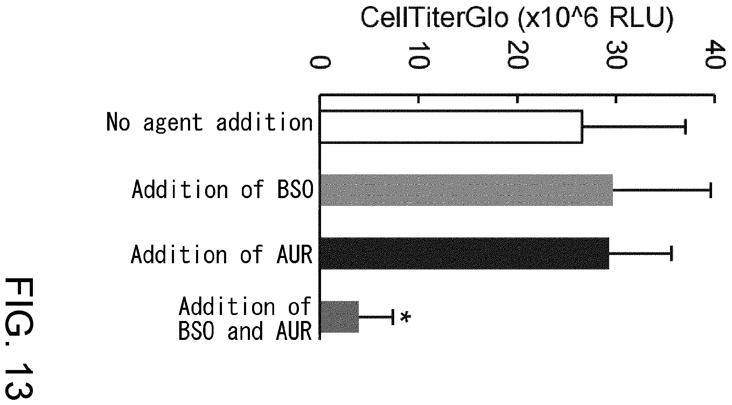

In order to confirm the contribution of GPX1 and GPX2 towards the regrowth of organoids, the gamma-glutamylcysteine synthetase inhibitor L-buthionine-sulfoximine (BSO, Cat # B2515, Merck, 200 µM) and the thioredoxin reductase inhibitor auranofin (AUR, Cat # A6733, Merck, 200 nM) were added upon re-culture in the re-culture method of Example 2-1, and cell proliferation was measured by CellTiter-Glo 3D Cell Viability Assay (Fig. 12). BSO inhibits the glutathione antioxidant pathway and AUR inhibits the thioredoxin antioxidant pathway, but organoid regrowth was suppressed only when both inhibitors were used in combination (n = 4, Fig. 13). From this result, it was suggested that not only the glutathione antioxidant pathway but also the thioredoxin pathway are involved in the regrowth of organoids, and that ROS metabolism is regulated by various mechanisms.

### [Industrial Applicability]

This time, a characteristic structure that occurs in cancer tissue of patients who received chemotherapy was found. The structure in the cancer tissue found by the present inventors had the characteristic phenotype of CSC. Furthermore, the present inventors succeeded in reconstructing *in vitro* the structure found in the cancer tissue this time. That is, the present disclosure has made it possible to artificially reconstruct the tissue structure discovered in cancer patients, which is found in the process of acquisition of anticancer agent resistance. The artificially reconstructed structure based on the present disclosure not only has the characteristics of the tissue structure generated in the process of acquiring resistance to an anticancer agent by a cancer, but also highly reproduces the gene expression profile. Therefore, the present disclosure makes it possible to observe the anticancer agent resistance acquiring process of a cancer using an artificially reconstructed tissue. Furthermore, the tissue constructed by the present disclosure is also useful as a tool for exploring new cancer therapeutic techniques targeting the anticancer agent resistance of cancers.

## Claims

1. A culture method for forming a treatment resistant organoid, wherein the method comprises a step of three-dimensionally culturing an organoid which was treated with at least one anticancer agent and isolating a treatment resistant organoid that is resistant to the treatment with said anticancer agent, wherein the step of three-dimensionally culturing is culturing in the absence of said at least one anticancer agent and wherein the organoid is formed by a pre-culture step of culturing one or more cancer stem cells.

2. The method according to claim 1, wherein the anticancer agent is one or more anticancer agents selected from agents that inhibit the growth of cancer cells.

3. The method according to claim 1 or 2, wherein the three-dimensional culture step comprises allowing the organoid which was treated with at least one anticancer agent to adhere to a first extracellular matrix and culturing by adding a first medium.

4. The method of any one of claims 1 to 3, wherein said treatment resistant organoid has at least one of the following histochemical characteristics:
(a) exhibits protrusions on the tissue surface composed of monolayer to multilayer cuboidal cells;
(b) is composed of large circular-oval cells containing cell debris in the organoid or lumen;
(c) exhibits compartmentalization, optionally wherein new glandular structures are formed; and
(d) is CD44 positive.

5. The method according to any one of claims 1 to 4, further comprising analyzing the gene expression level, protein expression level, protein modification, protein localization, epigenome, or metabolomics state of a cell comprised in the treatment resistant organoid.

6. The method according to any one of claims 1 to 4, comprising identifying an agent having a therapeutic activity against cancer, wherein the method further comprises:
(a) selecting at least one test agent;
(b) contacting the treatment resistant organoid with the test agent; and
(c) measuring the cytotoxic activity of the test agent against a cell comprised in the treatment resistant organoid or comparing the protein expression of a cell comprised in the treatment resistant organoid in the presence and absence of the agent.

7. The method according to claim 6 which is for identifying the mechanism of action of said agent having a therapeutic activity against cancer, wherein step (c) is comparing the protein expression of a cell comprised in the treatment resistant organoid in the presence and absence of the agent.

8. The method according to claim 6 which is for identifying a combination for combined use of an anticancer agent and said agent having a therapeutic activity against cancer, wherein step (c) is measuring the cytotoxic activity of the test agent against a cell comprised in the treatment resistant organoid.

## Patentansprüche

1. Kulturverfahren zur Bildung eines behandlungsresistenten Organoids, wobei das Verfahren einen Schritt des dreidimensionalen Kultivierens eines Organoids, das mit mindestens einem Antikrebsmittel behandelt wurde, und des Isolierens eines behandlungsresistenten Organoids, das resistent gegen die Behandlung mit dem Antikrebsmittel ist, umfasst, wobei der Schritt des dreidimensionalen Kultivierens ein Kultivieren in Abwesenheit des mindestens einen Antikrebsmittels ist und wobei das Organoid durch einen Vorkulturschritt des Kultivierens einer oder mehrerer Krebsstammzellen gebildet wird.

2. Verfahren nach Anspruch 1, wobei das Antikrebsmittel ein oder mehrere Antikrebsmittel ist, die ausgewählt sind aus Mitteln, die das Wachstum von Krebszellen inhibieren.

3. Verfahren nach Anspruch 1 oder 2, wobei der dreidimensionale Kulturschritt umfasst, zu ermöglichen, dass das Organoid, das mit mindestens einem Antikrebsmittel behandelt wurde, an einer ersten extrazellulären Matrix adhäriert und durch Hinzugabe eines ersten Mediums kultiviert wird.

4. Verfahren nach einem der Ansprüche 1 bis 3, wobei das behandlungsresistente Organoid mindestens eine der folgenden histochemischen Eigenschaften aufweist:
(a) zeigt Ausstülpungen auf der Gewebeoberfläche, die aus einschichtigen bis mehrschichtigen kubischen Zellen bestehen;
(b) besteht aus großen, kreisförmig-ovalen Zellen, die Zelltrümmer im Organoid oder Lumen enthalten;
(c) zeigt eine Kompartimentierung, gegebenenfalls wobei neue Drüsenstrukturen gebildet werden; und
(d) ist CD44-positiv.

5. Verfahren nach einem der Ansprüche 1 bis 4, des Weiteren umfassend das Analysieren des Genexpressionsspiegels, des Proteinexpressionsspiegels, der Proteinmodifikation, der Proteinlokalisierung, des Epigenoms oder des Metabolomzustands einer Zelle, die in dem behandlungsresistenten Organoid umfasst ist.

6. Verfahren nach einem der Ansprüche 1 bis 4, umfassend das Identifizieren eines Wirkstoffs, der eine therapeutische Aktivität gegen Krebs aufweist, wobei das Verfahren des Weiteren umfasst:
(a) Auswählen mindestens eines Testwirkstoffs;
(b) Inkontaktbringen des behandlungsresistenten Organoids mit dem Testwirkstoff; und
(c) Messen der cytotoxischen Aktivität des Testwirkstoffs gegenüber einer Zelle, die in dem behandlungsresistenten Organoid umfasst ist, oder Vergleichen der Proteinexpression einer Zelle, die in dem behandlungsresistenten Organoid umfasst ist, in Anwesenheit und Abwesenheit des Wirkstoffs.

7. Verfahren nach Anspruch 6, das zur Identifizierung des Wirkmechanismus des Wirkstoffs, der eine therapeutische Aktivität gegen Krebs aufweist, ist, wobei Schritt (c) das Vergleichen der Proteinexpression einer Zelle, die in dem behandlungsresistenten Organoid umfasst ist, in Anwesenheit und Abwesenheit des Wirkstoffs ist.

8. Verfahren nach Anspruch 6, das zur Identifizierung einer Kombination zur kombinierten Verwendung eines Antikrebsmittels und des Wirkstoffs, der eine therapeutische Aktivität gegen Krebs aufweist, ist, wobei Schritt (c) das Messen der cytotoxischen Aktivität des Testwirkstoffs gegenüber einer Zelle, die in dem behandlungsresistenten Organoid umfasst ist, ist.

## Revendications

1. Méthode de culture pour former un organoïde résistant à un traitement, laquelle méthode comprend une étape de culture tridimensionnelle d'un organoïde qui a été traité avec au moins un agent anticancéreux et d'isolation d'un organoïde résistant à un traitement qui est résistant au traitement avec ledit agent anticancéreux, dans laquelle l'étape de culture tridimensionnelle est une culture en l'absence dudit au moins un agent anticancéreux et dans laquelle l'organoïde est formé par une étape de préculture dans laquelle une ou plusieurs cellules souches cancéreuses sont cultivées.

2. Méthode selon la revendication 1, dans laquelle l'agent anticancéreux est un ou plusieurs agents anticancéreux choisis parmi les agents qui inhibent la croissance de cellules cancéreuses.

3. Méthode selon la revendication 1 ou 2, dans laquelle l'étape de culture tridimensionnelle comprend le fait de laisser l'organoïde qui a été traité avec au moins un agent anticancéreux adhérer à une première matrice extracellulaire et la culture par addition d'un premier milieu.

4. Méthode selon l'une quelconque des revendications 1 à 3, dans laquelle ledit organoïde résistant à un traitement a au moins une des caractéristiques histochimiques suivantes :
(a) il présente sur la surface tissulaire des protubérances composées de cellules cuboïdes monocouches ou multicouches ;
(b) il est composé de grosses cellules circulaires-ovales contenant des débris cellulaires dans l'organoïde ou la lumière ;
(c) il présente une compartimentalisation, éventuellement dans laquelle de nouvelles structures glandulaires sont formées ; et
(d) il est CD44 positif.

5. Méthode selon l'une quelconque des revendications 1 à 4, comprenant en outre l'analyse du niveau d'expression génique, du niveau d'expression protéique, de la modification protéique, de la localisation protéique, de l'épigénome, ou de l'état métabolomique d'une cellule comprise dans l'organoïde résistant à un traitement.

6. Méthode selon l'une quelconque des revendications 1 à 4, comprenant l'identification d'un agent ayant une activité thérapeutique contre un cancer, laquelle méthode comprend en outre :
(a) la sélection d'au moins un agent de test ;
(b) la mise en contact de l'organoïde résistant à un traitement avec l'agent de test ; et
(c) la mesure de l'activité cytotoxique de l'agent de test contre une cellule comprise dans l'organoïde résistant à un traitement ou la comparaison de l'expression protéique d'une cellule comprise dans l'organoïde résistant à un traitement en présence et en l'absence de l'agent.

7. Méthode selon la revendication 6, qui est destinée à l'identification du mécanisme d'action dudit agent ayant une activité thérapeutique contre un cancer, dans laquelle l'étape (c) est une comparaison de l'expression protéique d'une cellule comprise dans l'organoïde résistant à un traitement en présence et en l'absence de l'agent.

8. Méthode selon la revendication 6, qui est destinée à l'identification d'une combinaison pour une utilisation combinée d'un agent anticancéreux et dudit agent ayant une activité thérapeutique contre un cancer, dans laquelle l'étape (c) est une mesure de l'activité cytotoxique de l'agent de test contre une cellule comprise dans l'organoïde résistant à un traitement.
